# EUROPEAN PATENT SPECIFICATION

(11) **EP 1 446 165 B1**
(45) Date of publication and mention of the grant of the patent: **18.04.2007**
(21) Application number: 02779694.5
(22) Date of filing: 19.11.2002
(51) Int. Cl.: A61L 9/12, A61L 9/04, A01M 1/20, B65D 83/14

(54) **REFILL FOR AN AIR FRESHENER OR INSECTICIDE DEVICE**
NACHFÜLLPACKUNG FÜR EIN LUFTBELEBUNGS- ODER INSEKTIZIDGERÄT
CARTOUCHE DE REMPLISSAGE DE DISPOSITIF CONTENANT UN DESODORISANT D'ATMOSPHERE OU UN INSECTICIDE

(30) Priority: 20.11.2001 US 989294; 01.03.2002 US 86511
(43) Date of publication of application: 18.08.2004
(73) Proprietor: Reckitt Benckiser (UK) Limited, Slough, Berkshire SL1 3UH (GB)
(72) Inventor: RYMER, Shaun, Patrick, Hull HU8 7DS (GB); ROBINSON, Clare, Louise, Hull HU8 7DS (GB); McLEISH, Andrew, Sheffield S11 9AS (GB)
(74) Representative: Bowers, Craig Malcolm
(86) International application number: PCT/GB2002/005189
(87) International publication number: WO 2003/045450

(56) References cited:
- EP-A- 1 076 014
- US-A- 5 238 187

## Description

The present invention relates to a refill for a liquid air freshener or insecticidal device.

Refills are known in the art which comprise a cartridge containing a volatile liquid composition to be evaporated and a porous pad positioned, in use, beneath a lower face of the cartridge to receive the liquid composition as it is slowly released from the cartridge.

US-A-5238187 discloses a refill for a liquid air freshener comprising a volatile liquid cartridge, a porous sheet pad adapted to slowly receive liquid composition from the cartridge and a frame supporting the cartridge and sheet pad.

EP-A-1076014 discloses a dual-function dispenser which comprises a passive dispenser which is a twin gel cartridge dispenser comprising a pair of cartridge units connected end-to-end by a flexible hinge, adapted to engage the spray stem of a spray container.

The present invention provides an improvement to such refills. According to a first aspect of the present invention, there is provided a refill for a liquid air freshener or insecticidal device comprising a cartridge (1) containing a volatile liquid composition to be evaporated and a porous pad (5) positioned, in use, to receive the liquid composition as it is slowly released from the cartridge; wherein the porous pad is a part of a sheet (2) of material, the sheet of material and cartridge being supported in a frame (3), characterised in that the sheet of material is folded along at least one fold line (9) which, in use, extends substantially vertically to at least partially surround the cartridge.

Such an arrangement provides a simple way of securing the porous pad reliably in position beneath the cartridge which does not require a separate fitting operation by the consumer. It also provides a more substantial package which can be easily handled by the consumer and readily located with the housing of the air freshener or insecticidal device.

The provision of a pad with vertical fold lines has the advantage that the assembly of the sheet of material and frame is simply carried out by folding the sheet of material on a mandrel which folded material is then inserted into the frame. Complementary tags may be provided on the sheet and frame in order to guarantee precise location of the sheet within the frame. The folded sheet is retained in the frame by the resilience of the folded structure.

Preferably, the sheet of material comprises the porous pad, a rear panel and two side panels which are folded inwardly along the vertical fold lines towards one another from opposite sides of the rear panel. The porous pad may either be foldably attached to a bottom edge of the rear panel, or may be attached to one or both bottom edges of the side panels along a fold line which, in use, is substantially horizontal.

The frame and sheet may be provided as a sub-assembly wherein the frame has a foldable configuration with at least some of the fold lines corresponding to fold lines on the sheet. Alternatively, however, the frame has a non-foldable configuration as this lends itself to the manufacturing method with the mandrel described above.

According to a preferred aspect of the present invention the sheet of material is folded to meet the cartridge towards the end of the cartridge opposite to the end from which the composition is released.

By folding the sheet of material to meet the top end of the cartridge, the stability of the refill structure is enhanced as this top part supports the pad against the cartridge, and also can provide support for side panels of the sheet if these are present.

According to a further preferred aspect of the present invention the cartridge is retained in at least one pair of resilient jaws in the frame.

Providing a pair of resilient jaws to receive the cartridge provides a secure means of retaining the cartridge on the frame. Further, the manufacturing process is simplified as the cartridge is simply required to be moved in a single direction to ensure that it is retained on the frame. Preferably two pairs of resilient jaws are provided spaced along the length of the cartridge.

The present invention also extends to a method of manufacturing a refill for a liquid air freshener or insecticidal device comprising a cartridge containing a volatile liquid composition to be evaporated and a porous pad positioned, in use, to receive the liquid composition as it is slowly released from the cartridge; wherein the porous pad is a part of a sheet of material which is folded to at least partially surround the cartridge, the sheet of material and cartridge being supported in a frame; the method comprising the steps of: folding the porous pad over a mandrel; removing the mandrel from the folded pad; and inserting the folded pad into the frame, so that the folded pad is retained within the frame by the resilience of the folded structure.

Such a method provides a way of assembling the sheet of material into the frame which simply requires a two-stage process: folding on a mandrel and inserting into the frame. Preferably the frame is formed in a non-foldable configuration.

Preferably, the method further comprises the step of inserting the cartridge into the frame from the opposite direction to the direction in which the pad is inserted on the mandrel. This again facilitates the assembly procedure as the mechanisms for inserting the sheet of material and cartridge allow both to be inserted independently without interference from the other.

Preferably, the cartridge is retained in at least one pair of resilient jaws in the frame. Again, this facilitates insertion of the cartridge in a single direction.

This aspect of the method forms an independent aspect of the present invention which can broadly be defined as a method of making a refill for a liquid air freshener or insecticidal device comprising a cartridge containing a volatile liquid composition to be evaporated and a porous pad positioned, in use, to receive the liquid composition as it is slowly released from the cartridge; wherein the porous pad is a part of a sheet of material which is folded to at least partially surround the cartridge, the sheet of material and cartridge being supported in a frame; the method comprising the steps of: inserting the folded pad into the frame in a first direction; and inserting the cartridge into the frame in a second direction in which is opposite to the first direction.

Three examples of refills constructed in accordance with the present invention will now be described with reference to the accompanying drawings, in which:
Fig. 1A is an exploded view of a first example showing the assembly of the refill;
Fig. 1B is a perspective view of the assembled refill;
Fig. 2A is a view similar to Fig. 1A of a second example;
Fig. 2B is a view similar to Fig. 1B of a second example;
Figs. 3A and D are similar views to Figs 1A and 1B of a third example;
Fig. 3B is a perspective view of the folded sheet of the third example of Fig. 3A; and
Fig. 3C is a view similar to Fig. 3D without the cartridge.

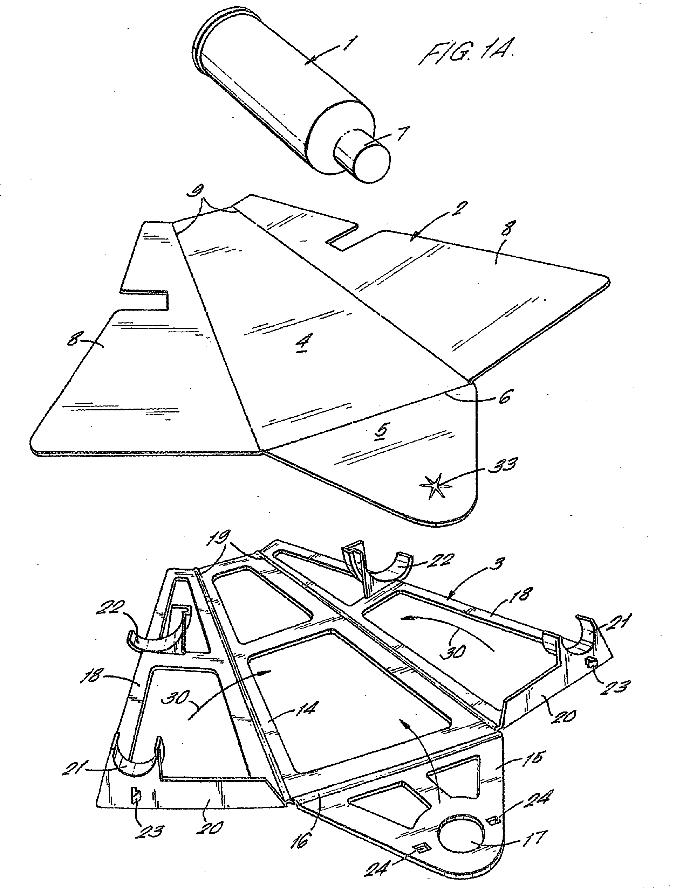

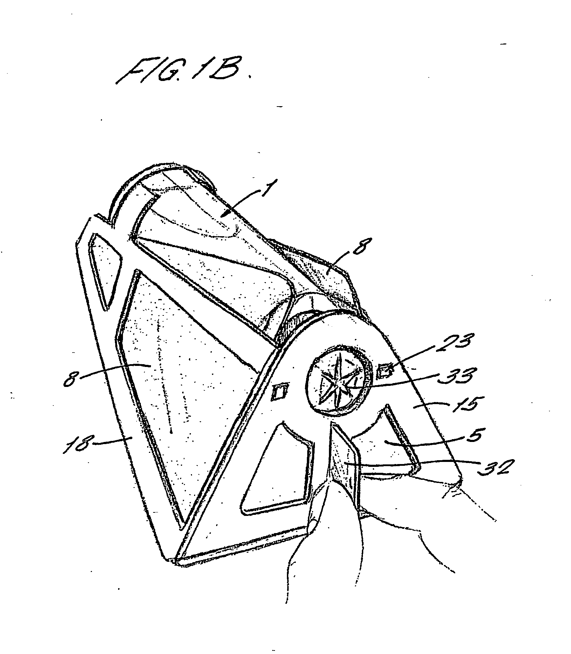

The first example will now be described with reference to Figs. 1A and 1B.

The refill comprises three components, a cartridge 1, a cardboard sheet 2, and a plastic frame 3.

The sheet 2 comprises a rear panel 4 with a base panel 5 attached along a fold line 6 to its lowermost edge. The base panel 5 provides the porous pad onto which, in use, liquid composition is gradually dispensed from the lower end 7 of cartridge 1. Two sheet side panels 8 are attached via fold lines 9 along the side edges of the rear panel 4.

The sheet 2 sits on the frame 3 which has a shape which generally conforms to the shape of the sheet 2. Thus, the frame 3 has a rear panel 14 corresponding in shape to the rear panel 4 of the sheet and a base panel 15 corresponding in shape to the base panel 5 of the sheet and attached to the rear panel 14 of the base along a fold line 16. The base panel 15 has an aperture 17 positioned to allow penetration of the lower end 7 of the cartridge 1. Two side panels 18 are attached along opposite sides of the rear panel 14 along fold lines 19. An upstanding flange 20 projects from the bottom edge of each side panel 18. A cartridge engaging jaw member 21 projects from the outer edge of the upstanding flange 20. A second jaw member 22 is provided towards the top of each side panel 18. A clip 23 extends from a downwardly facing surface of the upstanding flange 20. The clips 23 are arranged to engage in through holes 24 in the base panel 15.

To assemble the refill, the sheet 2 is positioned on the frame 3. The side panels 8/18 are then folded inwardly in the direction of arrows 30 until the cartridge 1 is held by jaw members 21 and 22. The base panel 5/15 is then folded upwardly in the direction of arrow 31 so that the clips 23 clip into the holes 24, and so that the lower end 7 of cartridge 1 is aligned with the aperture 17, thereby forming the finished refill as shown in Fig. 1B. The clips 23 penetrate the base panel 5 to assist in securing the sheet 2 in place.

As shown in Fig. 1B, a tab 32 is provided on the lower surface of the base panel 15 to allow the refill to be removed from the device housing. Further, a number of slits form a star-shaped aperture 33 in the base panel 5. This aperture 33 is arranged to be pierced by a spike in the housing into which the refill is placed. The spike also pierces the lower end 7 of the cartridge 1 to allow flow of the composition out of the cartridge 1 thereby initiating operation of the device.

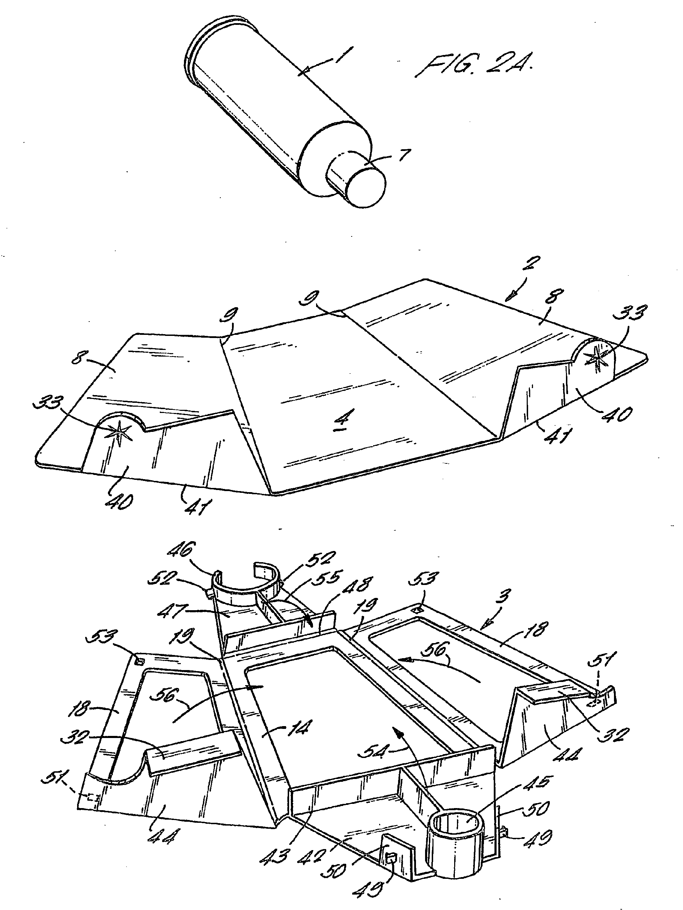

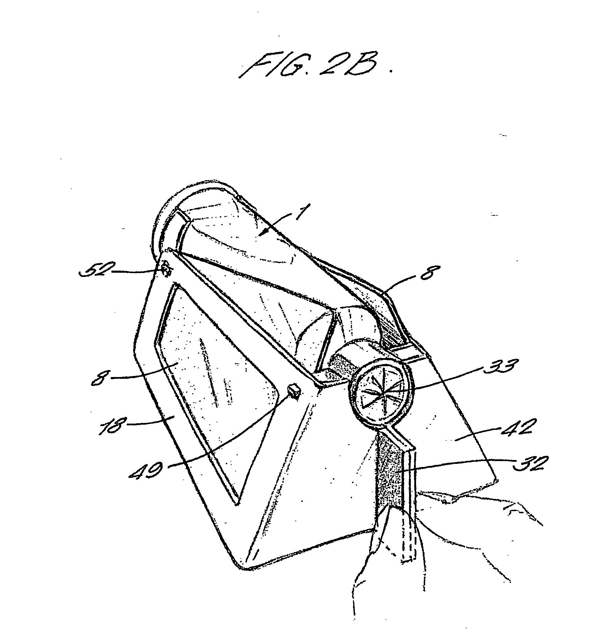

The second example will now be described with reference to Figs. 2A and 2B. Broadly, this has the same construction as the first example with the cartridge 1, cardboard sheet 2 and plastic frame 3. The sheet 2 has a similar rear panel 4 and two adjacent side panels 8 joined to the rear panel along fold lines 9. The frame 3 has a similar rear panel 14 and adjacent side panels 18 joined to the rear panel along fold lines 19.

However, in place of the base panel 5 of the first example, the sheet 2 is provided with a pair of base panels 40, each base panel being attached along a fold line 41 to a lowermost edge of a respective side panel 8. The base panels 40 are half the size of the base panel 5 of the first example and are arranged to meet one another when the refill is folded up to provide a similar configuration to the base panel 5 of the first example. Each base panel 40 has a portion with the star-shaped orifice 33. In the assembled refill, these portions will overlap forming a porous pad of double thickness beneath the lower end 7 of the cartridge 1.

The frame 3 has a base panel 42 attached to the lowermost edge of the rear panel 14 via a fold line 43. Additional parts of the base are provided by a pair of half panels 44 which extend from the lowermost edges of respective side panels 18 and are arranged to support the base panels 40 of the sheet 2. Each half panel 44 has a tab 32 positioned so that tabs 32 abut one another in the assembled refill as shown in Fig. 2B.

The jaw member 21 of the first example is replaced by a cylindrical orifice 45 which is open at its bottom end as shown in Fig. 2B to allow the spike to penetrate when the refill is inserted into the housing. The upper jaw members 22 of the first example are replaced by a single jaw member 46 which is a part circular jaw member attached to the top of an upper panel 47 which is attached to the upper edge of the rear panel 14 along a fold line 48.

To clip the frame together, a pair of clips 49 are provided on flanges 50 which extend upwardly from an outer edge of the base panel 42. These engage with corresponding openings 51 in the side panels 18. A second pair of clips 52 are provided on the top panel 47. These engage with a second pair of openings 53 in the side panels 18.

With the sheet 3 in place on the frame 3, the base panel 42 is folded up in the direction of arrow 54 so that the bottom end of cartridge 1 is retained in cylindrical orifice 45. The top panel 47 is folded downwardly in the direction of arrow 55 so that the top of the cartridge 1 is retained within jaw member 46. The side panels 18 are then folded inwardly in the direction of arrows 56 bringing the tabs 32 together, and bringing the star-shaped orifices 33 into overlapping relation beneath the lower end 7 of the cartridge 1. At the same time, the clips 49 and 52 clip into their respective holes 51 and 53 securing the frame in place. The clips penetrate through the cardboard sheet 2 and also serve to secure this in place. The assembled refill can then be handled as described with reference to the first example.

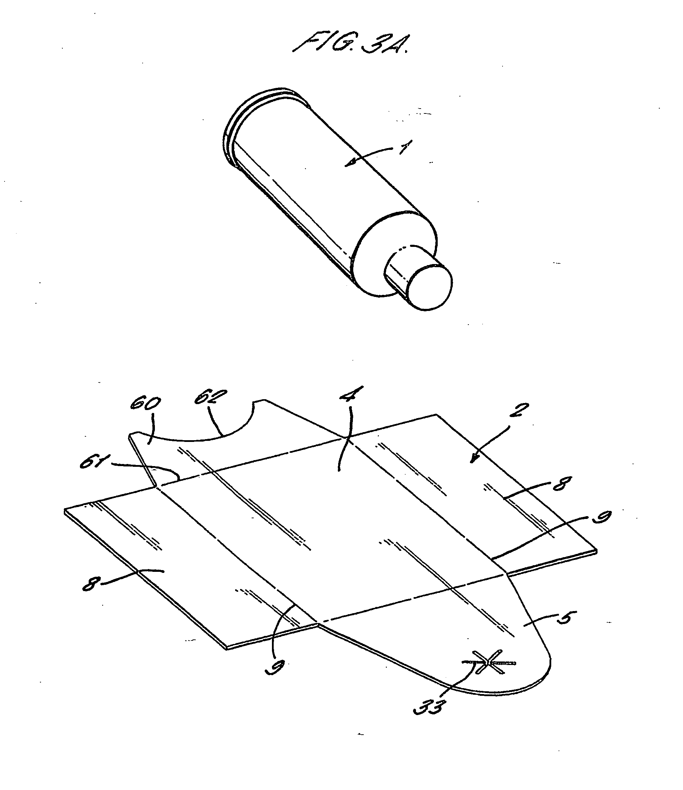

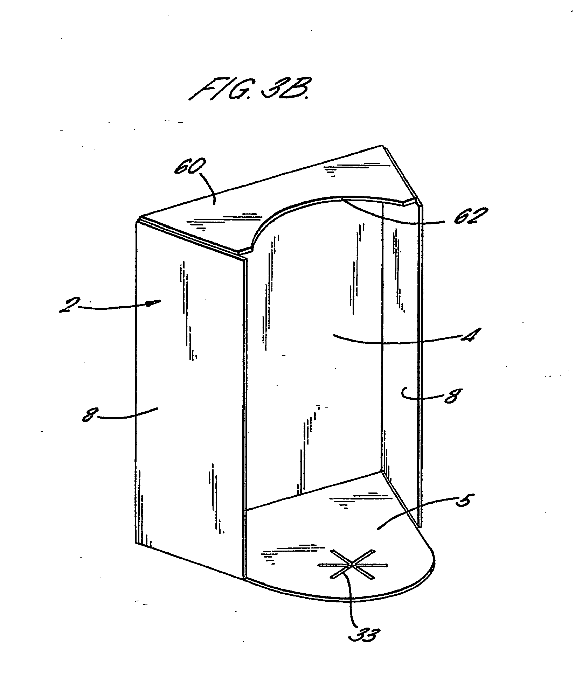

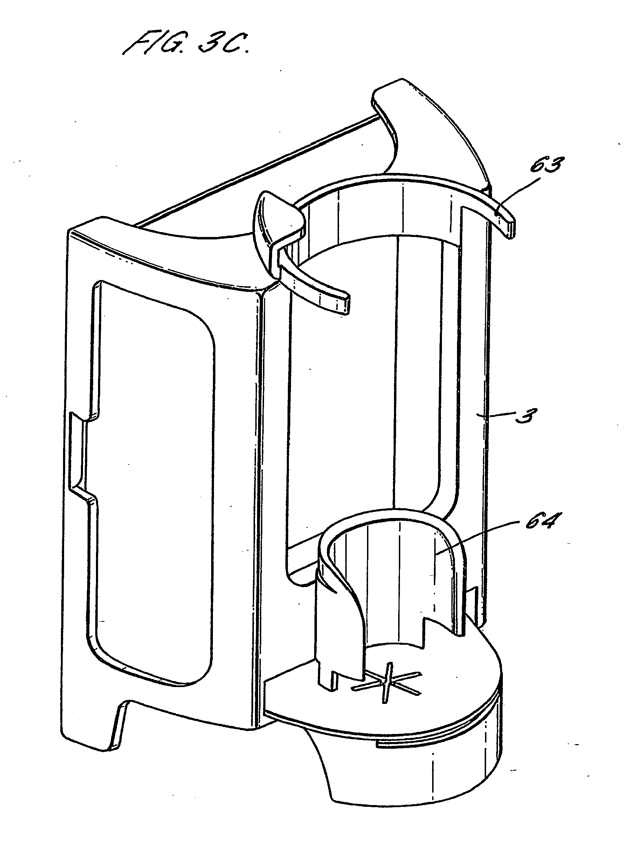

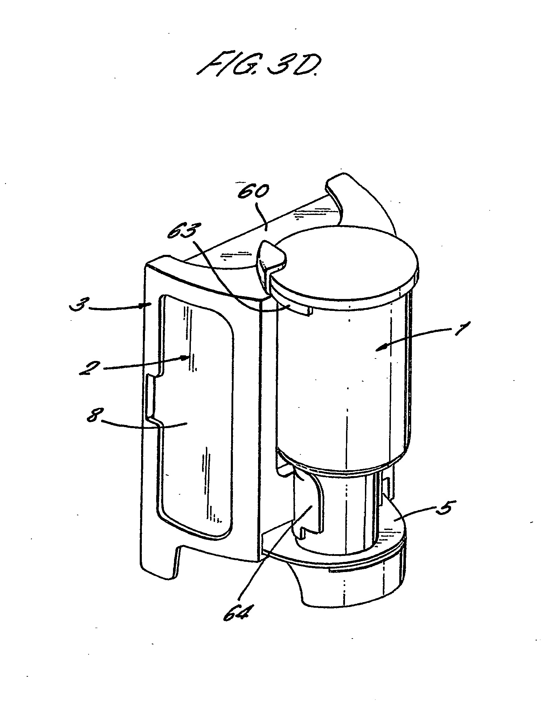

The third example will now be described with reference to Figs. 3A, 3B and 3C. Again this has broadly the same construction as the first and second examples with the cartridge 1, cardboard sheet 2 and plastic frame 3. The sheet 2 has a similar rear panel 4 and two adjacent side panels 8 joined to the rear panel along fold lines 9. The sheet 2 has a similar base panel 5 to that of the first example, and in addition has a single end panel 60 which is attached to the rear panel 4 along the fold line 61. The end panel 60 has an arcuate cut out 62, to accommodate the outer wall of the cartridge 1, when the sheet is in position within the frame 3.

The frame 3 differs from the frame of the second example in that it is a non-foldable frame which is not provided in an assembleable form. It is manufactured as a moulding in a preassembled form as shown in Fig. 3C. The sheet 2 is folded on a mandrel along each of the fold lines to form the structure as shown in Fig. 3B. The mandrel is a metal block with an end face shaped generally in the shape of the rear panel 4. The mandrel is then removed from the folded sheet manually and the folded sheet is inserted manually into an open underside of the frame 3. The resilience of the folded sheet retains it in place in the frame. The base panel 5 is supported by the frame 3 in a position such that the lower end of the cartridge 7 will rest on or very close thereto, when it is inserted in the frame 3. The cartridge 1 is inserted into the frame 3 so that it is supported by clip members 63, 64 at each end of the frame 3 in the correct position relative to the base panel 5. The assembled refill can then be handled as described above, with reference to the first example.

## Claims

1. A refill for a liquid air freshener or insecticidal device comprising a cartridge (1) containing a volatile liquid composition to be evaporated and a porous pad (5) positioned, in use, to receive the liquid composition as it is slowly released from the cartridge; wherein the porous pad is a part of a sheet (2) of material, the sheet of material and cartridge being supported in a frame (3), **characterised in that** the sheet of material is folded along at least one fold line (9) which, in use, extends substantially vertically to at least partially surround the cartridge.

2. A refill according to claim 1, wherein the sheet of material comprises the porous pad (5), a rear panel (4) and two side panels (8) which are folded inwardly along the vertical fold lines towards one another from opposite sides of the rear panel.

3. A refill according to any one of the preceding claims, wherein the folded sheet is retained in the frame by the resilience of the folded structure.

4. A refill according to claim 1 wherein the sheet of material is folded to meet the cartridge towards the end of the cartridge opposite to the end from which the composition is released.

5. A refill according to claim 2 wherein the cartridge is retained in at least one pair of resilient jaws (21,22) in the frame.

6. A method of manufacturing a refill for a liquid air freshener or insecticidal device comprising a cartridge containing a volatile liquid composition to be evaporated and a porous pad positioned, in use, to receive the liquid composition as it is slowly released from the cartridge; wherein the porous pad is a part of a sheet of material which is folded to at least partially surround the cartridge, the sheet of material and cartridge being supported in a frame; the method comprising the steps of: folding the porous pad over a mandrel; removing the mandrel from the folded pad; and inserting the folded pad into the frame, so that the folded pad is retained within the frame by the resilience of the folded structure.

7. A method according to claim 6, further comprising the step of inserting the cartridge into the frame from the opposite direction to the direction in which the pad is inserted on the mandrel.

8. A method according to claim 6, further comprising the steps of: inserting the folded pad into the frame in a first direction; and inserting the cartridge into the frame in a second direction in which is opposite to the first direction.

9. A method according to claim 8, wherein the cartridge is retained in at least one pair of resilient jaws in the frame.

## Patentansprüche

1. Nachfüllung für eine flüssige Luftreiniger- oder Insektizidvorrichtung, umfassend eine Patrone (1), enthaltend eine zu verdampfende flüchtige, flüssige Zusammensetzung und ein poröses Polster (5), das bei Verwendung derart positioniert ist, dass es die flüssige Zusammensetzung aufnimmt, wenn sie aus der Patrone langsam freigesetzt wird; wobei das poröse Polster ein Teil einer Materiallage (2) ist, wobei die Materiallage und die Patrone in einem Rahmen (3) gehalten werden, **dadurch gekennzeichnet, dass** die Materiallage entlang mindestens einer Faltlinie (9) gefaltet ist, die sich bei Verwendung im Wesentlichen senkrecht erstreckt, um die Patrone zumindest teilweise zu umgeben.

2. Nachfüllung nach Anspruch 1, wobei die Materiallage das poröse Polster (5), eine hintere Platte (4) und zwei Seitenplatten (8), die entlang der senkrechten Faltlinien zueinander von gegenüber liegenden Seiten der hinteren Platte nach innen gefaltet sind.

3. Nachfüllung nach einem der vorangehenden Ansprüche, wobei die gefaltete Lage durch die Nachgiebigkeit der gefalteten Struktur im Rahmen gehalten wird.

4. Nachfüllung nach Anspruch 1, wobei die Materiallage derart gefaltet ist, dass sie zum Ende der Patrone hin gegenüber dem Ende, von welchem die Zusammensetzung freigesetzt wird, auf die Patrone trifft.

5. Nachfüllung nach Anspruch 2, wobei die Patrone in mindestens einem Paar nachgiebiger Klemmbacken (21, 22) im Rahmen gehalten wird.

6. Verfahren zur Herstellung einer Nachfüllung für eine flüssige Luftreiniger- oder Insektizidzusammensetzung, umfassend eine Patrone, enthaltend eine zu verdampfende flüchtige, flüssige Zusammensetzung und ein poröses Polster (5), das bei Verwendung derart positioniert ist, dass es die flüssige Zusammensetzung aufnimmt, wenn sie aus der Patrone langsam freigesetzt wird, wobei das poröse Polster ein Teil einer Materiallage ist, die derart gefaltet ist, dass sie zumindest teilweise die Patrone umgibt und die Materiallage und die Patrone in einem Rahmen gehalten werden, umfassend die folgenden Schritte: Falten des porösen Polsters um einen Aufspanndorn; Entfernen des Aufspanndorns von dem gefalteten Polster; und Einsetzen des gefalteten Polsters in den Rahmen derart, dass das gefaltete Polster durch die Nachgiebigkeit der gefalteten Struktur im Rahmen gehalten wird.

7. Verfahren nach Anspruch 6, des Weiteren umfassend den Schritt des Einsetzens der Patrone in den Rahmen aus der zur Richtung, in welchem das Polster auf den Aufspanndorn eingesetzt wird, gegensätzlichen Richtung.

8. Verfahren nach Anspruch 6, des Weiteren umfassend die folgenden Schritte: Einsetzen des gefalteten Polsters in den Rahmen in einer ersten Richtung; und Einsetzen der Patrone in den Rahmen in einer zweiten Richtung, die gegensätzlich zur ersten Richtung ist.

9. Verfahren nach Anspruch 8, wobei die Patrone in mindestens einem Paar nachgiebiger Klemmbacken im Rahmen gehalten wird.

## Revendications

1. Recharge pour un dispositif contenant un désodorisant ou un insecticide liquide, comprenant une recharge (1) contenant une composition de liquide volatil prévue pour évaporation et un tampon poreux (5) positionné, en utilisation, pour recevoir la composition de liquide à mesure qu'elle est libérée lentement de la recharge ; dans laquelle le tampon poreux fait partie d'une feuille (2) de matériau, la feuille de matériau et la recharge étant supportées dans un bâti (3), **caractérisée en ce que** la feuille de matériau est pliée le long d'au moins une ligne (9) de pliage qui, en utilisation, s'étend sensiblement verticalement pour au moins entourer partiellement la recharge.

2. Recharge selon la revendication 1, dans laquelle la feuille de matériau comprend le tampon poreux (5), un panneau arrière (4) et deux panneaux latéraux (8) qui sont pliés, l'un vers l'autre, vers l'intérieur le long des lignes verticales de pliage par rapport aux côtés opposés du panneau arrière.

3. Recharge selon l'une quelconque des revendications précédentes, dans laquelle la feuille pliée est maintenue dans le bâti par l'élasticité de la structure pliée.

4. Recharge selon la revendication 1, dans laquelle la feuille de matériau est pliée pour rencontrer la recharge en direction de l'extrémité de la recharge opposée à l'extrémité à partir de laquelle la composition est libérée.

5. Recharge selon la revendication 2, dans laquelle la recharge est maintenue dans au moins une paire de mâchoires élastiques (21, 22) situées dans le bâti.

6. Procédé de fabrication d'une recharge de remplissage pour un dispositif contenant un désodorisant ou un insecticide liquide, comprenant une recharge contenant une composition de liquide volatil prévue pour évaporation et un tampon poreux positionné, en utilisation, pour recevoir la composition de liquide à mesure qu'elle est libérée lentement de la recharge ; dans laquelle le tampon poreux fait partie d'une feuille de matériau qui est pliée pour au moins entourer partiellement la recharge, la feuille de matériau et la recharge étant supportées dans un bâti ; procédé comprenant les étapes, dans lesquelles: on plie le tampon poreux sur un mandrin ; on retire le mandrin du tampon plié ; et l'on introduit le tampon plié dans le bâti, de sorte que le tampon plié est maintenu à l'intérieur du bâti par l'élasticité de la structure pliée.

7. Procédé selon la revendication 6, comprenant en outre l'étape d'introduction de la recharge dans le bâti dans le sens opposé au sens dans lequel le tampon est introduit sur le mandrin.

8. Procédé selon la revendication 6, comprenant en outre les étapes dans lesquelles : on introduit le tampon plié dans le bâti dans un premier sens ; et l'on introduit la recharge dans le bâti dans un second sens qui est opposé au premier sens.

9. Procédé selon la revendication 8, dans lequel la recharge est maintenue dans au moins une paire de mâchoires élastiques situées dans le bâti.
